(19) Europäisches Patentamt
European Patent Office
Office européen des brevets

(11) Veröffentlichungsnummer: **0 104 551**
**B1**

(12) # EUROPÄISCHE PATENTSCHRIFT

(45) Veröffentlichungstag der Patentschrift: **13.11.85**

(51) Int. Cl.⁴: **C 07 C 119/045**, C 08 G 18/75

(21) Anmeldenummer: **83109106.1**

(22) Anmeldetag: **15.09.83**

(54) **Gegebenenfalls Isomerengemische darstellende cycloaliphatische Diisocyanate, ein Verfahren zu ihrer Herstellung, sowie ihre Verwendung als Ausgangsmaterialien bei der Herstellung von Polyurethankunststoffen.**

(30) Priorität: **25.09.82 DE 3235573**

(43) Veröffentlichungstag der Anmeldung:
**04.04.84 Patentblatt 84/14**

(45) Bekanntmachung des Hinweises auf die Patenterteilung:
**13.11.85 Patentblatt 85/46**

(84) Benannte Vertragsstaaten:
**BE DE FR GB IT**

(56) Entgegenhaltungen:
**EP - A - 0 024 665**
**EP - A - 0 046 917**
**DE - A - 1 768 832**
**DE - A - 2 361 986**
**US - A - 3 180 883**

**Houben-Weyl, Methoden der organischen Chemie, Bd. 1/1 (1950) S. 356-357**
**Bayer-Kunststoffe 3e Ausgabe (1963), S.43**
**Beilsteins Handbuch der organischen Chemie 4e Aufl. (1930), Bd. 213 S. 238, 254**

(73) Patentinhaber: **BAYER AG, Konzernverwaltung RP Patentabteilung, D-5090 Leverkusen 1 Bayerwerk (DE)**

(72) Erfinder: **Knöfel, Hartmut, Dr., Duelmener Weg 21, D-5068 Odenthal (DE)**
Erfinder: **Brockelt, Michael, Neuenhauser Weg 1, D-5060 Bergisch-Gladbach (DE)**
Erfinder: **Penninger, Stefan, Dr., Pommernallee 5, D-4047 Dormagen 1 (DE)**
Erfinder: **Stutz, Herbert, Dr., Schulstrasse 81, D-4047 Dormagen 5 (DE)**

**EP 0 104 551 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue, nur an einem Cyclohexanring mono- oder dialkylsubstituierte Methylen-bis-(cyclohexylisocyanate), ein Verfahren zu ihrer Herstellung durch Phosgenierung der ihnen zugrundeliegenden Diamine bzw. Diamingemische und ihre Verwendung als Ausgangsmaterial zur Herstellung von Polyurethankunststoffen nach dem Isocyanat-Polyadditionsverfahren.

Aliphatische oder cycloaliphatische Diisocyanate, wie Hexamethylendiisocyanat, 3,5,5-Trimethyl-1-isocyanato-3-isocyanatomethyl-cyclohexan und 4,4'-Methylen-bis-(cyclohexylisocyanat) sowie deren Stellungs- und/oder Stereoisomerengemische werden in der Polyurethanchemie z. B. zur Herstellung von lichtechten Beschichtungsmaterialien mit guter Witterungsbeständigkeit verwendet. Letztere lassen sich außerdem durch Umsetzung mit Polyolen gut zu Lackbindemitteln, Elastomeren oder Schaumstoffen verarbeiten, falls das Diisocyanat bei Raumtemperatur flüssig und mit den Polyolen verträglich bzw. ausreichend mischbar ist (vgl. z. B. DE-OS 1 768 832). Reines trans, trans-4,4'-Methylen-bis-(cyclohexylisocyanat) ist beispielsweise für diese Zwecke nicht geeignet, da es bei Raumtemperatur fest ist (Schmp.: 83°C), eine geringe Löslichkeit in Polyolen aufweist und sich deshalb vor Beendigung der Polyadditionsreaktion durch Kristallisation dem Reaktionsgemisch entzieht. Auch das aus trans, trans-, cis, trans- und cis, cis-4,4'-Methylen-bis-(cyclohexylisocyanat) bestehende Stereoisomerengemisch, wie es nach Kernhydrierung von 4,4'-Diaminodiphenylmethan und anschließender Phosgenierung anfällt, ist bei Raumtemperatur fest, da bei der Hydrierung unter normalen Bedingungen das trans, trans-Diamin als Hauptprodukt anfällt, und somit zur Herstellung der obengenannten Polyurethane nur bedingt einsetzbar.

Die Abtrennung von trans, trans-4,4'-Methylen-bis-(cyclohexylisocyanat) aus dem Stereoisomerengemisch ist zwar technisch möglich z. B. durch Fällung der trans, trans-Isomere als Carbamidsäurechlorid und anschließende Filtration (vgl. JA-PS 119 844), jedoch wird durch diesen zusätzlichen Verfahrensschritt die Isocyanatausbeute vermindert, was als großer Nachteil anzusehen ist.

Flüssige cycloaliphatische Diisocyanate können nach dem Stand der Technik durch Phosgenierung von 2,4'-Methylen-bis-(cyclohexylisocyanat) hergestellt werden (vgl. DE-OS 1 768 832). Dieser Offenlegungsschrift zufolge sind Gemische von 2,4'- und 4,4'-Isomeren auch dann flüssig, wenn der 2,4'-Isomerenanteil 30 bis 95 Gew.-% beträgt und das 4,4'-Isomere einen trans, trans-Isomerenanteil von weniger als 50 Gew.-% besitzt. Es erwies sich jedoch als Nachteil, daß das 2,4'-Isomere in reiner Form nur schwer zugänglich ist, da es in drei Stufen durch Kondensation von Anilin mit Formaldehyd, Kernhydrierung und Phosgenierung hergestellt wird und beim Kondensationsschritt 2,4'-Diaminodiphenylmethan selbst unter optimalen Bedingungen nur mit ca. 30% der Theorie anfällt (vgl. DE-OS 1 937 685), d. h. zur Isolierung des reinen 2,4'-Isomeren ist eine technisch aufwendige Isomerentrennung vonnöten.

Die direkte Herstellung eines 2,4'-isomerenreichen Methylen-bis-(cyclohexylisocyanats) durch Phosgenierung eines entsprechenden Methylen-bis-(cyclohexylamin)-Isomerengemisches ist ebenfalls mit Nachteilen behaftet, da bei der Anilin/Formaldehyd-Kondensation der Gehalt des 2,4'-Diaminodiphenylmethans mit dem des 2,2'-Isomeren korelliert und letzteres bei der Kernhydrierung einer Zersetzungsreaktion unterliegt.

Es war deshalb die Aufgabe der vorliegenden Erfindung neue cycloaliphatische Diisocyanate auf Basis von Methylen-bis-(cyclohexylisocyanat) aufzufinden, die bei Raumtemperatur flüssig sind, in bezug auf Löslichkeit und Verträglichkeit mit Polyolen den Forderungen der Praxis genügen, und die nicht mit den Nachteilen des Standes der Technik behaftet sind.

Diese Aufgabe konnte überraschenderweise durch die Bereitstellung der nachfolgend näher beschriebenen Diisocyanate bzw. Diisocyanatgemische und des zu ihrer Herstellung geeigneten Verfahrens gelöst werden.

Gegenstand der Erfindung sind, gegebenenfalls Isomerengemische darstellende Diisocyanate der allgemeinen Formel

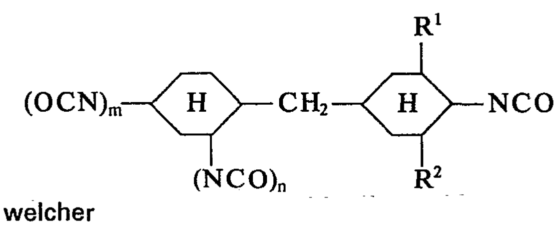

in welcher

$R^1$ und $R^2$ jeweils gleich oder verschieden sind und Wasserstoff oder (gegebenenfalls verzweigte) Alkylgruppen mit 1 bis 12, vorzugsweise 1 bis 4 Kohlenstoffatomen, insbesondere Methylgruppen bedeuten, mit der Maßgabe, daß mindestens einer der Reste $R^1$ und $R^2$ für einen Alkylrest steht und

m und n jeweils für 0 oder 1 stehen, mit der Maßgabe, daß die Summe $m+n$ den Wert 1 ergibt, wobei im Falle von m oder $n=0$ die verbleibende freie Valenz durch Wasserstoff abgesättigt ist.

Gegenstand der Erfindung ist auch ein Verfahren zur Herstellung dieser Diisocyanate oder Diisocyanatgemische, welches dadurch gekennzeichnet ist, daß man die ihnen zugrundeliegenden, gegebenenfalls als Stellungs- und/oder Stereoisomerengemisch vorliegenden cycloaliphatischen Diaminen in an sich bekannter Weise phosgeniert.

Gegenstand der Erfindung ist schließlich auch die Verwendung der neuen Diisocyanate bzw. Diisocyanatgemische als Aufbaukomponente bei der Herstellung von Polyurethankunststoffen nach dem Isocyanat-Polyadditionsverfahren.

Ausgangsmaterialien für das erfindungsgemäße Verfahren zur Herstellung der neuen Diisocyanate sind, gegebenenfalls als Stellungs- und/oder Stereoisomerengemische vorliegende cycloaliphatische Diamine der allgemeinen Formel

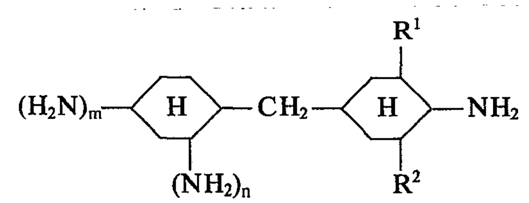

in welcher

$R^1$, $R^2$, m und n die bereits obengenannte Bedeutung haben.

Ihre Herstellung erfolgt vorzugsweise durch Kernhydrierung der ihnen zugrundeliegenden Diaminodiphenylmethane.

Diese asymmetrisch substituierten Diaminodiphenylmethane können beispielsweise durch Kondensation von o- und/oder p-Nitrobenzylhalogeniden, insbesondere -chloriden, mit substituierten Anilinen der Formel

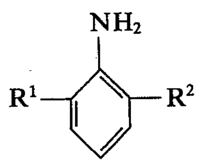

Reduktion der Nitrogruppe zum primären Amin und anschließende Umlagerung in Analogie zu BE-PS 864 533 oder GB-PS 1 567 114 erhalten werden. Je nach dem, ob bei der Kondensationsreaktion reines o-Nitrobenzylhalogenid bzw. reines p-Nitrobenzylhalogenid bzw. Gemische dieser Isomeren eingesetzt werden, entstehen letztendlich beim erfindungsgemäßen Verfahren reine Isomere oder Isomerengemische darstellende Diisocyanate der bereits obengenannten allgemeinen Formel, wobei das Verhältnis der 4,4'-Diisocyanato-3(,5)-(di)-alkyldiphenylmethane zu den 4,2'-Diisocyanato-3(,5)-(di)-alkyldiphenylmethanen weitgehend dem bei der Kondensationsreaktion eingesetzten Isomerenverhältnis o-Nitrobenzylhalogenid : p-Nitrobenzylhalogenid entspricht (lediglich bei Verwendung von monosubstituierten Anilinen ($R^2 = H$) entstehen untergeordnete Mengen (bis zu 5 Gew.-%, bezogen auf Gesamtgemisch) an 2,4'-Diisocyanato-3-alkyldiphenylmethanen und/oder an dem entsprechenden 2,2'-Isomeren über die Zwischenstufe der entsprechenden Diamine).

Die Kernhydrierung der aromatischen Diamine erfolgt im allgemeinen nach den bekannten Methoden des Standes der Technik (vgl. P. Rylander, Catalytic Hydrogenation in Organic Syntheses, Academic Press New York, San Francisco, London (1979), S. 190). Hierbei werden die aromatischen Diamine bis zur vollständigen Wasserstoffaufnahme katalytisch hydriert. Die Hydrierung erfolgt bei 20 bis 300°C unter einem Druck von 20 bis 300 bar, vorzugsweise bei einer Temperatur von 100 bis 300°C, insbesondere von 150 bis 250°C und bei einem Druck von 70 bis 300 bar, insbesondere 120 bis 250 bar.

Die Hydrierung erfolgt in Gegenwart von 0,1 bis 30 Gew.-%, vorzugsweise von 0,1 bis 10 Gew.-%, bezogen auf katalytisch wirksames Metall einerseits und Diaminoverbindung andererseits, eines Hydrierungskatalysators. Geeignete Katalysatoren sind beispielsweise, gegebenenfalls auf inerten Trägern wie Aktivkohle, Kieselgel und insbesondere Aluminiumoxid vorliegende Elemente der 8. Nebengruppe des Periodensystems der Elemente oder katalytisch wirksame anorganische Verbindungen dieser Elemente. Besonders gut geeignet sind z. B. Ruthenium-, Platin-, Rhodium-, Nickel- und/oder Kobaltkatalysatoren in elementarer oder chemisch gebundener Form. Besonders bevorzugt werden Ruthenium oder katalytisch wirksame Rutheniumverbindungen eingesetzt. Beispiele geeigneter Rutheniumverbindungen sind Rutheniumdioxid, Rutheniumtetroxid, Bariumperruthenit, Natrium-, Kalium-, Silber-, Calcium- oder Magnesiumruthenat, Natriumperruthenat, Rutheniumpentafluorid, Rutheniumtetrafluoridhydrat oder Rutheniumtrichlorid. Falls Trägersubstanzen für die Katalysatoren mitverwendet werden, beträgt der Metallgehalt des Trägerkatalysators im allgemeinen 1 bis 10 Gew.-%, vorzugsweise 1 bis 5 Gew.-%. Im übrigen ist selbstverständlich die Art und Menge des einzusetzenden Katalysators keineswegs wesentlich.

Es ist oft zweckmäßig, die Hydrierungsreaktion in Gegenwart von Ammoniak durchzuführen, da Ammoniak unerwünschte Desaminierungsreaktionen und die Bildung von sekundären Aminen als Nebenprodukte unterdrückt. Falls Ammoniak mitverwendet wird, geschieht dies in Mengen von 0,1 bis 30 Gew.-%, vorzugsweise 5 bis 10 Gew.-%, bezogen auf zu hydrierende Ausgangsmaterialien.

Die Hydrierung kann lösungsmittelfrei oder in Gegenwart inerter Lösungsmittel durchgeführt werden. Im allgemeinen werden niedrigschmelzende bzw. flüssige aromatische Diamine in Substanz, hochschmelzende Diamine dagegen in gelöster Form hydriert. Als Lösungsmittel eignen sich unter den Reaktionsbedingungen inerte organische Verbindungen mit niedrigem Siedepunkt, vorzugsweise Alkohole wie Methanol, Ethanol, n-Propanol, i-Propanol oder Ether wie z. B. Dioxan, Tetrahydrofuran oder Diethylether oder Kohlenwasserstoffe wie Cyclohexan. Die Durchführung der Hydrierung findet kontinuierlich in einem Reaktionsrohr, einer Druckkesselkaskade oder vorzugsweise diskontinuierlich in einem Rührautoklaven statt, indem man den Autoklaven mit Katalysator, der zu hydrierenden Substanz und gegebenenfalls einem Lösungsmittel beschickt, mehrmals mit Inertgas spült und gegebenenfalls Ammoniak zudosiert. Danach drückt man Wasserstoff auf, bringt das Gemisch auf Reaktionstemperatur, hydriert bis Druckkonstanz erreicht ist und rührt während eines weiteren Zeitraums von ca. 0,5 bis 5 Stunden bei gleicher Temperatur. Nach Abkühlung des Reaktionsgemisches und Abtrennung des Katalysators wird das Hydrierungsprodukt im allgemeinen destillativ aufgearbeitet.

Die Hydrierungsprodukte fallen mit hohen Ausbeuten, die im allgemeinen über 90% der Theorie liegen, an und können durch Destillation von Nebenprodukten wie z. B. Amino-alkylbenzyl-cyclohexylamin befreit werden. Sie stellen im allgemeinen Stereo-, gegebenenfalls auch Stellungsisomerengemische dar und entsprechen bezüglich der Stellungsisomerie weitgehend den Ausgangsmaterialien. Eine Auftrennung in einzelne Stellungs- und/oder Stereoisomere ist im allgemeinen bezüglich der technischen Verwendbarkeit der Hydrierungsprodukte als Ausgangsmaterialien für das erfindungsgemäße Verfahren nicht erforderlich, da es hierbei keineswegs auf Isomerenreinheit ankommt und im Gegenteil häufig Isomerengemische erwünscht sind, da dies die Eigenschaften der Diisocyanate verbessert.

Bevorzugte beim erfindungsgemäßen Verfahren einzusetzende, gemäß dem beschriebenen Hydrierungsverfahren zugängliche Ausgangsmaterialien sind beispielsweise 4,4'-Diamino-3,5-dimethyldicyclohexylmethan, 4,4'-Diamino-3,5-diethyldicyclohexylmethan, 4,4'-Diamino-3,5-diisopropyldicyclohexylmethan, 4,4'-Diamino-3-ethyl-5-methyldicyclohexylmethan, sowie diese Diamine als wesentliche Komponente enthaltende Isomerengemische; 4,2'-Diamino-3,5-dimethyldicyclohexylmethan, 4,2'-Diamino-3,5-diethyldicyclohexylmethan, 4,2'-Diamino-3,5-diisopropyldicyclohexylmethan, 4,2'-Diamino-3-ethyl-5-methyldicyclohexylmethan, sowie diese Diamine als wesentliche Komponente enthaltende Isomerengemische; 4,4'-Diamino-3-methyldicyclohexylmethan, 4,4'-Diamino-3-ethyldicyclohexylmethan, 4,4'-Diamino-3-isopropyldicyclohexylmethan und deren Gemische mit anderne Stellungsisomeren, wie z. B. den entsprechenden 4,2'-Diamino-3-alkyldiphenylmethanen und/oder 2,4'-Diamino-3-alkyldiphenylmethanen, 4,2'-Diamino-3-methyldicyclohexylmethan, 4,2'-Diamino-3-ethyldicyclohexylmethan, 4,2'-Diamino-3-isopropyldicyclohexylmethan sowie deren Gemische mit den entsprechenden 4,4'-Diamino-3-alkyl- bzw. 2,4'-Diamino-3-alkyldicyclohexylmethanen.

Bei der Durchführung des erfindungsgemäßen Verfahrens zur Herstellung der neuen Diisocyanate erfolgt die Phosgenierung der beispielhaft genannten Diamine oder deren Salze nach an sich bekannten Verfahren in Gegenwart eines inerten organischen Lösungsmittels (vgl. Houben—Weyl, Methoden der organischen Chemie, Georg Thieme Verlag Stuttgart (1952), Band 8, 4. Auflage, Seiten 120 ff).

Als zu phosgenierende Salze eignen sich vorzugsweise die Hydrochloride oder Ammoniumcarbamate, die durch Sättigung der Diaminlösungen mit gasförmigem Chlorwasserstoff bzw. Kohlendioxid anfallen. Prinzipiell können auch andere Salze, die beispielsweise durch Neutralisation der Diamine mit Protonen abspaltenden Säuren anfallen, phosgeniert werden.

Die Selektivität der Phosgenierungsreaktion ist weitgehend von der Aminkonzentration und vom Phosgenüberschuß abhängig. Vorzugsweise wird das Phosgen in einem hohen molaren Überschuß und das zu phosgenierende Diamin in stark verdünnter Form eingesetzt. Im allgemeinen beträgt der molare Phosgenüberschuß 100 bis 2000%, vorzugsweise 100 bis 1000% und die Aminkonzentration in der zur Phosgenierung gelangenden Aminlösung 0,1 bis 15 Gew.-%, vorzugsweise 5 bis 10 Gew.-%.

Als Lösungsmittel können beliebige inerte organische Flüssigkeiten oder deren Gemische mit einem Siedepunkt von 60—250° C, d. h. Halogenkohlenwasserstoffe, Aromaten, Hydroaromaten sowie deren Chlorverbindungen verwendet werden. Als Beispiele geeigneter Lösungsmittel seien Xylol, Mesitylen, Chlorbenzol, Dichlorbenzol, Trichlorbenzol, Chlornaphthalin und Dichlorethan genannt.

Die Reaktion wird entweder einstufig durch Heißphosgenierung bei Temperaturen von 100 bis 250° C oder zweistufig durch Kalt/Heißphosgenierung bei Temperaturen von —20° C bis 250° C unter Normaldruck durchgeführt.

Bei Verwendung der freien Amine als Ausgangsverbindung (Basenphosgenierung) wird zuerst bei Temperaturen von —20 bis +60° C Ammoniumcarbamidsäurechlorid hergestellt, das dann bei Temperaturen von 20 bis 250° C mit Phosgen zum Diisocyanat weiterreagiert.

Eine bevorzugte Ausführungsform des Verfahrens besteht darin, die Amine in einem geeigneten organischen Lösungsmittel zu lösen, diese durch Einleiten von Kohlendioxid als Ammoniumcarbamate zu fällen und die Suspension bei 0—50° C mit der theoretischen Menge Phosgen zu behandeln.

Daraufhin wird die Temperatur unter weiterem Einleiten von Phosgen langsam gesteigert bis bei Temperaturen von 100 bis 180° C eine klare Lösung entsteht.

Die Reinigung der Verfahrensprodukte erfolgt im allgemeinen nach Entphosgenierung durch Abdampfen des Lösungsmittels und anschl. Destillation unter vermindertem Druck.

Die erfindungsgemäßen Verfahrensprodukte, d. h. die neuen erfindungsgemäßen Diisocyanate fallen in hohen Ausbeuten als farblose, niedrigviskose Flüssigkeiten an und stellen wertvolle Aufbaukomponenten bei der Herstellung von Polyurethankunststoffen nach dem Isocyanat-Polyadditionsverfahren dar. Die Stellungs- und/oder Stereoisomerie der neuen Diisocyanate entspricht der Isomerie der bei der Phosgenierung eingesetzten Diamine. Im allgemeinen ist es nicht erforderlich, die beim erfindungsgemäßen Verfahren anfallenden Gemische in einzelne Stellungs- und/oder Stereoisomere aufzutrennen, da die erfindungsgemäßen Verfahrensprodukte ohne derartige Trennungsoperationen direkt der erfindungsgemäßen Verwendung zugeführt werden können. Hierin ist einer der Hauptvorteile der erfindungsgemäßen Diisocyanate bzw. Diisocyanatgemische gegenüber den unsubstituierten Methylen-bis-(cyclohexylisocyanaten) des Standes der Technik zu sehen. Die neuen erfindungsgemäßen Diisocyanate können besonders vorteilhaft zur Herstellung von Polyurethanlacken, Polyurethanelastomeren oder Polyurethanschaumstoffen eingesetzt werden, wobei sie bei den an sich bekannten Verfahren zur Herstellung derartiger Kunststoffe anstelle oder zusammen mit den bislang eingesetzten Polyisocyanaten zum Einsatz gelangen.

Die nachfolgenden Beispiele dienen der weiteren Erläuterung der Erfindung. Alle Prozentangaben beziehen sich, soweit nicht anders lautendes vermerkt, auf Gewichtsprozente.

## Beispiel 1

a) In einem 0,7 l Edelstahlautoklaven werden 250 g (1,18 Mol) 4,4'-Diamino-3-methyldiphenylmethan in Gegenwart von 25 g $Ru/Al_2O_3$-Trägerkatalysator (5% Ru) und 25 g Ammoniak bei 180° C und 200 bar während 4 Stunden hydriert. Nach Abtrennung des Katalysators wird das Produkt durch Flash-Destillation vorgereinigt und über eine Vigreux-Kolonne fraktioniert destilliert. Es werden 256,2 g (97% der Theorie) mit einem Siedepunkt von 110 bis 112° C/0,3 mbar an 4,4'-Diamino-3-methyldicyclohexylmethan gewonnen.

b) In eine Lösung von 112 g (0,5 Mol) 4,4'-Diamino-3-methyldicyclohexylmethan in 1,4 l getrocknetem Chlorbenzol wird bei Raumtemperatur unter gutem Rühren trockenes Kohlendioxid eingeleitet, bis keine Aufnahme mehr stattfindet. Die entstandene Suspension wird auf 30° C gekühlt und mit 200 g Phosgen behandelt wobei die Temperatur unter Viskositätsabnahme auf 50° C steigt. Nun bringt man das Reaktionsgemisch innerhalb von 2 Stunden auf Rückfluß und leitet dabei 300 g Phosgen ein. Man kocht noch 4 Stunden unter weiterem Einleiten von Phosgen, sowie eine Stunde unter Einleiten von Stickstoff. Danach wird das Lösungsmittel durch Vakuumdestillation abgetrennt und das Rohprodukt bei 168—170° C/0,2—0,4 mbar destilliert.

Man erhält 114,6 g (80% der Theorie) 4,4'-Diisocyanato-3-methyldicyclohexylmethan mit einem NCO-Gehalt von 29,3%, einem Gehalt an hydrolysierbarem Chlor von 0,06% und einer Viskosität bei 25° C von 30 mPas.

## Beispiel 2

a) In Analogie zu Beispiel 1a) werden 250 g (0,98 Mol) 4,4'-Diamino-3,5-diethyldiphenylmethan in Gegenwart von 25 g Ruthenium-Trägerkatalysator und 25 g Ammoniak bei 200° C und 200 bar bis zur vollständigen Wasserstoffaufnahme hydriert. Man läßt auf Raumtemperatur abkühlen, entspannt, löst das Rohprodukt in Methanol auf und filtriert den Katalysator ab. Danach wäscht man zweimal mit Methanol nach, vereinigt die Lösungen und destilliert das Rohprodukt nach Abdampfen des Lösungsmittels bei 110—112° C/0,1 mbar. Die Ausbeute an 4,4'-Diamino-3,5-diethyldicyclohexylmethan beträgt 236,4 g.

b) Man löst 133 g (0,5 Mol) des gemäß Beispiel 2a) hergestellten Diamins in 1,4 l getrocknetem Chlorbenzol und leitet unter intensivem Rühren trockenes Kohlendioxid bis zur Sättigung ein. Dabei bildet sich ein fluoreszierender Niederschlag und das Gemisch erwärmt sich auf 60—70° C. Man kühlt auf Raumtemperatur ab begast die Suspension mit 200 g Phosgen und heizt unter Einleiten von 150 g/h Phosgen langsam auf Rückflußtemperatur. Der Niederschlag agglomeriert bei 65—70° C zu einer schwer rührbaren Masse, die oberhalb von 75° C langsam schmilzt. Man phosgeniert die bei 130° C entstehende klare Lösung weitere 2 Stunden, stoppt den Phosgenstrom und leitet statt dessen Stickstoff ein, um überschüssiges Phosgen zu vertreiben. Nun wird zuerst das Lösungsmittel bei 1000—100 mbar und anschließend 4,4'-Diisocyanato-3,5-diethyl-dicyclohexylmethan bei 162—165° C/0,3—0,4 mbar destilliert. Die Ausbeute beträgt 140,7 g (87,8% der Theorie), NCO-Gehalt: 26,2%, hydrolysierbares Chlor: 0,02%, Viskosität/25° C: 110 mPas.

Beispiel 3

a) Man hydriert entsprechend Beispiel 1a) 250 g 4,4'-Diamino-3,5-diisopropyldiphenylmethan in Gegenwart von 25 g $Ru/Al_2O_3$-Katalysator und 25 g Ammoniak bei 200° C und 200 bar. Nach Abtrennung des Katalysators und destillativer Aufarbeitung bei 141—144° C/0,1 mbar werden 235,3 g 4,4'-Diamino-3,5-diisopropyldicyclohexylmethan gewonnen.

b) Davon werden 147 g (0,5 Mol) in 1,4 l vorgetrocknetem Chlorbenzol gelöst und bei Raumtemperatur trockenes Kohlendioxid eingeleitet. Nach Sättigung der entstehenden Suspension leitet man 200 g Phosgen ein. Das Reaktionsgemisch erwärmt sich auf 60° C und der Niederschlag agglomeriert zu einer zähen Masse. Durch rasches Heizen und weiteres Einleiten von 150 g/h Phosgen bringt man den Feststoff zum Schmelzen und es entsteht bei 125° C eine klare Lösung. Man phosgeniert weitere 3 Stunden bei konstanten Bedingungen, befreit die Lösung durch Stickstoffspülung von überschüssigem Phosgen und destilliert Chlorbenzol ab. Das Rohprodukt wird durch rasche Destillation bei 162—164° C/0,1 mbar gereinigt. Dabei werden 158,4 g (91,6% der Theorie) 4,4'-Diisocyanato-3,5-diisopropyldicyclohexylmethan (NCO-Gehalt: 23,6%, hydrolysierbares Chlor: 0,03%, Viskosität/25° C: 460 mPas) gewonnen.

Beispiel 4

a) 250 g 4,4'-Diamino-3,5-dimethyldiphenylmethan werden in Gegenwart von 25 g $Ru/Al_2O_3$ und 25 g Ammoniak gemäß Beispiel 1a) bei 200° C und 200 bar hydriert. Nach Aufarbeitung und Destillation bei 118—122° C/0,1 mbar gewinnt man 235,5 g der perhydrierten Ausgangsverbindung.

b) Eine Lösung von 119 g (0,5 Mol) 4,4'-Diamino-3,5-dimethyldicyclohexylmethan in 1,4 l trockenem Clorbenzol wird unter Rühren mit Kohlendioxid gesättigt und die entstehende Suspension bei 30—50° C mit 200 g Phosgen behandelt. Man heizt innerhalb von 2 Stunden unter Einleiten von 150 g/h Phosgen auf 120° C, wobei der Niederschlag vollständig in Lösung geht und phosgeniert noch weitere 3 Stunden. Danach wird eine Stunde entphosgeniert, das Lösungsmittel abdestilliert und das Rohprodukt einer Flashdestillation bei 138—140° C/0,1 mbar unterworfen. Dabei werden 123,5 g (83,4% der Theorie) 4,4'-Diisocyanato-3,5-dimethyldicyclohexylmethan (NCO-Gehalt: 28,4% hydrolysierbares Chlor: 0,05%, Viskosität/25° C: 50 mPas) isoliert.

Beispiel 5

a) Man hydriert gemäß Beispiel 1a) 250 g 4,4'-Diamino-3-ethyl-5-methyl-diphenylmethan in Gegenwart von 25 g eines handelsüblichen Rutheniumkatalysators (5 Gew.-% Ruthenium auf $Al_2O_3$) und 25 g Ammoniak. Das Rohprodukt wird in Methanol aufgenommen, der Katalysator abfiltriert, gewaschen und die vereinigten Lösungen destilliert. Bei einer Temperatur von 130—131° C und einem Druck von 0,1 mbar gehen 251,3 g 4,4'-Diamino-3-ethyl-5-methyldicyclohexylmethan über.

b) Eine Lösung von 126 g (0,5 Mol) 4,4'-Diamino-3-ethyl-5-methyldicyclohexylmethan in 1,4 l Chlorbenzol wird mit trockenem Kohlendioxid gesättigt und die entstehende Suspension bei Temperaturen von 30 bis 50° C unter intensivem Rühren mit 200 g Phosgen versetzt. Man erhitzt das Reaktionsgemisch unter weiterem Einleiten von Phosgen bis zum Sieden, wobei der Feststoff vollständig in Lösung geht, und phosgeniert 3 Stunden nach. Danach wird entphosgeniert, das Lösungsmittel abgedampft und das Rohprodukt bei 145—147° C/0,05 mbar destilliert. Dabei fallen 129,3 g (83,5% der Theorie) 4,4'-Diisocyanato-3-ethyl-5-methyldicyclohexylmethan (NCO-Gehalt: 27,1% hydrolysierbares Chlor: 0,03%, Viskosität/25° C: 70 mPas) an.

Beispiel 6

a) Man beschickt einen 0,7 l Rührautoklaven mit 212 g (1 Mol) 4,4'-Diamino-3-methyldiphenylmethan und 21,2 g Ruthenium-Aluminiumoxid-Trägerkatalysator (5% Ru), spült mehrmals mit Stickstoff und Wasserstoff und dosiert 21,2 g Ammoniak zu. Danach wird unter intensivem Rühren bei 200° C und 200 bar bis zur vollständigen Wasserstoffaufnahme hydriert. Nach Abkühlen und Entspannen des Autoklaven nimmt man das Produkt in Methanol auf, saugt vom Katalysator ab und wäscht den Katalysator mehrmals mit Methanol. Die vereinigten Lösungen werden destillativ aufgearbeitet.

Bei einer Siedetemperatur von 127° C/0,15 mbar gehen 204,9 g (91% der Theorie) 4,2'-Diamino-3-methyldicyclohexylmethan über.

b) Man löst 150 g (0,67 Mol) 4,2'-Diamino-3-dicyclohexylmethan in 2 l wasserfreiem Chlorbenzol und leitet bei 40 bis 50° C Kohlendioxid bis zur Sättigung ein. Daraufhin kühlt man die entstandene Suspension auf 10° C ab und leitet in raschem Strom unter Rühren und Kühlen 250 g Phosgen ein. Das Reaktionsgemisch wird nun unter mäßigem Einleiten von Phosgen innerhalb von 1 Stunde auf

Rückflußtemperatur erhitzt und weitere 90 Minuten unter gleichen Bedingungen nachphosgeniert. Man entphosgeniert und reinigt das Diisocyanat durch zweimalige Destillation unter vermindertem Druck. Die Ausbeute beträgt 165 g (89% der Theorie) 4,2'-Diisocyanato-3-methyl-dicyclohexylmethan, das durch folgende Daten charakterisiert ist:

| | |
|---|---|
| Siedepunkt | 110—120° C/0,05 mbar |
| NCO-Wert | 30,0% |
| Viskosität | 30 mPa · s/25° C |
| Gehalt an hydrolysierbarem Chlor | 0,02% |

Beispiel 7

a) In einem 0,7 l Rührautoklaven hydriert man 226 g (1 Mol) 4,2'-Diamino-3,5-dimethyldiphenylmethan in Gegenwart von 22,6 g Ruthenium-Aluminiumoxid-Trägerkatalysator (5% Ru) und 22,6 g Ammoniak bei 200° C und 200 bar bis zur Druckkonstanz. Das Produkt wird in Methanol aufgelöst, der Katalysator abgesaugt, mit Methanol gewaschen und die vereinigten Lösungen destilliert. Als Ausbeute erhält man 209,8 g (88% Der Theorie) 4,2'-Diamino-3,5-dimethyldicyclohexylmethan, das einen Siedepunkt von 99 bis 105° C/0,015 mbar besitzt.

b) 150 g (0,63 Mol) 4,2'-Diamino-3,5-dimethyldicyclohexylmethan werden in 2 l wasserfreiem Chlorbenzol gelöst und mit Kohlendioxid begast. Es bildet sich ein Feststoff, der bei 10°C unter intensivem Rühren mit 250 g Phosgen zur Reaktion gebracht wird. Man heizt unter mäßigem Einleiten von Phosgen auf 125° C auf und rührt weitere 6 Stunden nach, bis der Feststoff gelöst ist. Danach wird entphosgeniert und das Produkt mittels Destillation gereinigt. Man erhält 163 g (89% der Theorie) 4,4'-Diisocyanato-3,5-dimethyldicyclohexylmethan mit einem Siedepunkt von 115—125° C/0,03 mbar, einen NCO-Gehalt von 26,7%, einen Gehalt an hydrolysierbarem Chlor von 0,01% und einer Viskosität von 44 mPa · s/25° C.

**Patentansprüche**

1. Gegebenenfalls Isomerengemische darstellende Diisocyanate der allgemeinen Formel

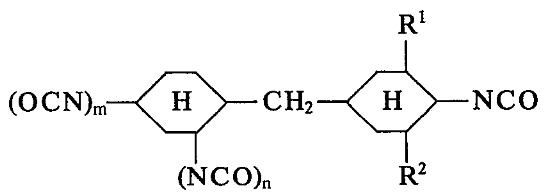

in welcher

$R^1$ und $R^2$ jeweils gleich oder verschieden sind und Wasserstoff oder (gegebenenfalls verzweigte) Alkylgruppen mit 1 bis 12 Kohlenstoffatomen bedeuten mit der Maßgabe, daß mindestens einer der Reste $R^1$ und $R^2$ für einen Alkylrest steht und

m und n jeweils für 0 oder 1 stehen, mit der Maßgabe, daß die Summe $m+n$ den Wert 1 ergibt, wobei im Falle von m oder $n=0$ die verbleibende freie Valenz durch Wasserstoff abgesättigt ist.

2. Diisocyanate gemäß Anspruch 1 der allgemeinen Formel

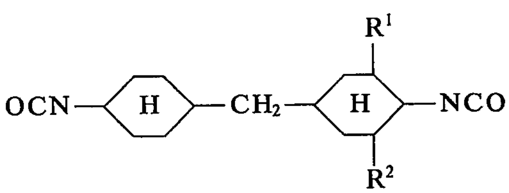

dadurch gekennzeichnet, daß

$R^1$ und $R^2$ für gleiche oder verschiedene Reste stehen und jeweils (gegebenenfalls verzweigte) Alkylgruppen mit 1 bis 4 Kohlenstoffatomen bedeuten.

3. Diisocyanate gemäß Anspruch 1 der allgemeinen Formel

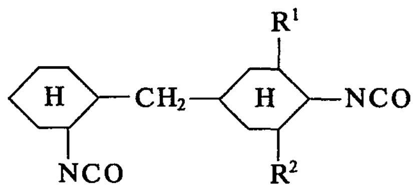

dadurch gekennzeichnet, daß

$R^1$ und $R^2$ für gleiche oder verschiedene Reste stehen und jeweils (gegebenenfalls verzweigte) Alkylgruppen mit 1 bis 4 Kohlenstoffatomen bedeuten.

4. Diisocyanate gemäß Anspruch 1 der allgemeinen Formel

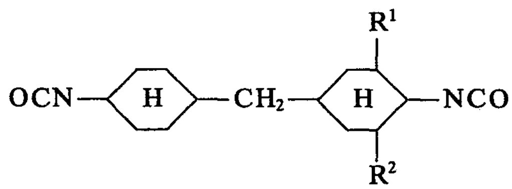

dadurch gekennzeichnet, daß

$R^1$ eine (gegebenenfalls verzweigte) Alkylgruppe mit 1 bis 4 Kohlenstoffatomen bedeutet und
$R^2$ für Wasserstoff steht.

5. Diisocyanate gemäß Anspruch 1 der allgemeinen Formel

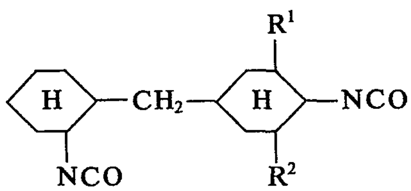

dadurch gekennzeichnet, daß

$R^1$ eine (gegebenenfalls verzweigte) Alkylgruppe mit 1 bis 4 Kohlenstoffatomen bedeutet und
$R^2$ für Wasserstoff steht.

6. Verfahren zur Herstellung von Diisocyanaten oder Diisocyanatgemischen gemäß Anspruch 1 bis 5, dadurch gekennzeichnet, daß man die ihnen zugrundeliegenden, gegebenenfalls als Stellungs- und/oder Stereoisomerengemisch vorliegenden cycloaliphatischen Diaminen in an sich bekannter Weise phosgeniert.

7. Verfahren gemäß Anspruch 6, dadurch gekennzeichnet, daß man die Phosgenierungsreaktion bei einer Temperatur von $-20$ bis $+250°$ C durchführt.

8. Verfahren gemäß Anspruch 6 und 7, dadurch gekennzeichnet, daß man als zu phosgenierende Diamine oder Diamingemische die Hydrierungsprodukte der ihnen konstitutionell entsprechenden aromatischen Diamine verwendet.

9. Verwendung der Diisocyanate gemäß Anspruch 1 bis 5 als Ausgangsmaterialien bei der Herstellung von Polyurethankunststoffen nach dem Isocyanat-Polyadditionsverfahren.

## Claims

1. Diisocyanates, optionally in the form of isomer mixtures, of the general formula

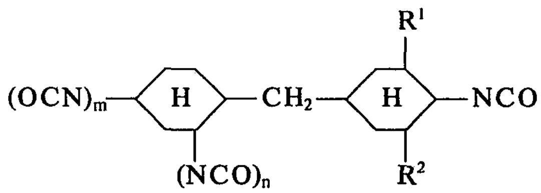

in which

$R^1$ and $R^2$ are identical or different in each case and denote hydrogen or (optionally branched) alkyl groups with 1 to 12 carbon atoms, with the proviso that at least one of the radicals $R^1$ and $R^2$ represents an alkyl radical, and

m and n each represent 0 or 1, with the proviso that the sum of $m+n$ is 1, and in the case where m or $n=0$ the remaining free valency is saturated by hydrogen.

2. Diisocyanates according to claim 1 of the general formula

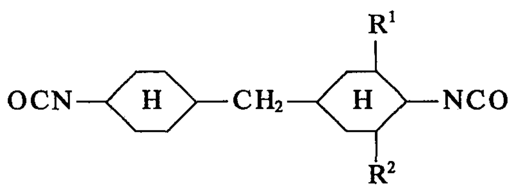

characterised in that

$R^1$ and $R^2$ represent identical or different radicals and each denote (optionally branched) alkyl groups with 1 to 4 carbon atoms.

3. Diisocyanates according to claim 1 of the general formula

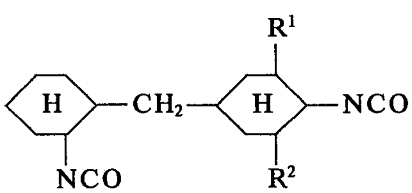

characterised in that

$R^1$ and $R^2$ represent identical or different radicals and each denote (optionally branched) alkyl groups with 1 to 4 carbon atoms.

4. Diisocyanates according to claim 1 of the general formula

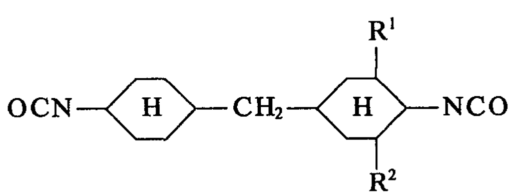

characterised in that

$R^1$ denotes an (optionally branched) alkyl group with 1 to 4 carbon atoms and
$R^2$ represents hydrogen.

5. Diisocyanates according to claim 1 of the general formula

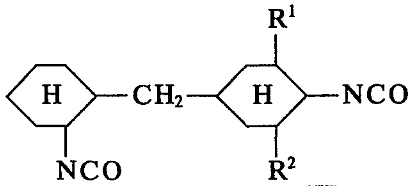

characterised in that

$R^1$ denotes an (optionally branched) alkyl group with 1 to 4 carbon atoms and
$R^2$ represents hydrogen.

6. Process for the production of diisocyanates or diisocyanate mixtures according to claim 1 to 5, characterised in that the cycloaliphatic diamines on which they are based and which are optionally present in form of a mixture of position and/or stereoisomers are phosgenated in a manner known per se.

7. Process according to claim 6, characterised in that the phosgenation reaction is carried out at a temperature of −20 to +250° C.

8. Process according to claim 6 and 7, characterised in that the diamines or diamine mixtures used for phosgenation are the hydrogenation products of the constitutionally corresponding aromatic diamines.

9. Use of the diisocyanates according to claim 1 to 5 as starting materials in the production of polyurethane plastics by the isocyanate polyaddition process.

**Revendications**

1. Diisocyanates représentant éventuellement des mélanges d'isomères, de formule générale

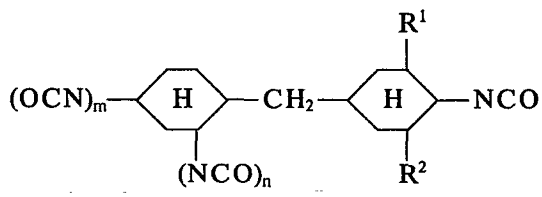

dans laquelle

$R^1$ et $R^2$ sont égaux ou différents et représentent chacun l'hydrogène ou des groupes alkyle (éventuellement ramifiés) ayant 1 à 12 atomes de carbone, sous réserve qu'au moins l'un des restes $R^1$ et $R^2$ représente un reste alkyle et
m et n sont tous deux égaux à 0 ou à 1, sous réserve que la somme m + n donne la valeur 1, et lorsque m ou n est égal à 0, la valence libre restante est saturée par de l'hydrogène.

2. Diisocyanates suivant la revendication 1, de formule générale

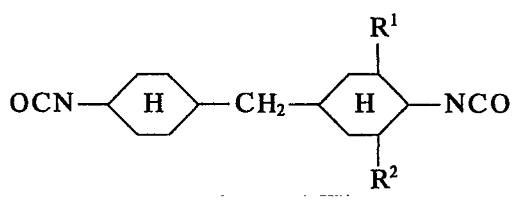

caractérisés en ce que

$R^1$ et $R^2$ représentent des restes égaux ou différents et désignent chacun des groupes alkyle (éventuellement ramifiés) ayant 1 à 4 atomes de carbone.

3. Diisocyanates suivant la revendication 1, de formule générale

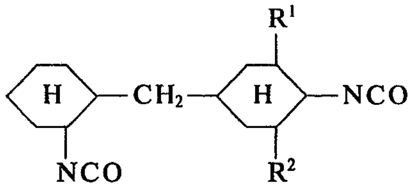

caractérisés en ce que

$R^1$ et $R^2$ sont des restes égaux ou différents et représentent chacun des groupes alkyle (éventuellement ramifiés) ayant 1 à 4 atomes de carbone.

4. Diisocyanates suivant la revendication 1, de formule générale

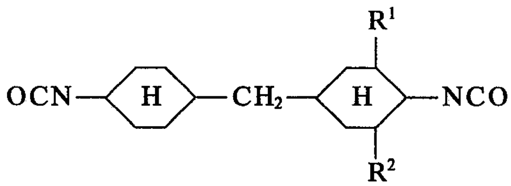

caractérisés en ce que

$R^1$ représente un groupe alkyle (éventuellement ramifié) ayant 1 à 4 atomes de carbone et
$R^2$ est l'hydrogène.

5. Diisocyanates suivant la revendication 1, de formule générale

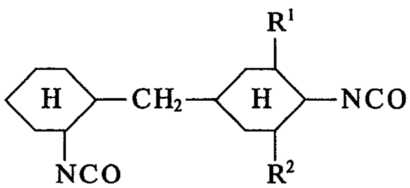

caractérisés en ce que

$R^1$ est un groupe alkyle (éventuellement ramifié) ayant 1 à 4 atomes de carbone et
$R^2$ désigne l'hydrogène.

6. Procédé de production de diisocyanates ou de mélanges de diisocyanates suivant les revendications 1 à 5, caractérisé en ce qu'on phosgène d'une manière connue les diamines cycloaliphatiques qui en constituent la base et qui se présentent éventuellement sous la forme d'un mélange d'isomères de position et/ou de stéréoisomères.

7. Procédé suivant la revendication 6, caractérisé en ce que la réaction de phosgénation est conduite à une température de $-20$ à $+250°$ C.

8. Procédé suivant les revendications 6 et 7, caractérisé en ce qu'on utilise comme diamines ou comme melanges de diamines à phosgéner, les produits d'hydrogénation des diamines aromatiques qui y correspondent par leur constitution.

9. Utilisation des diisocyanates suivant les revendications 1 à 5 comme matières de départ dans la production de matières plastiques du type polyuréthanne par le procédé de polyaddition d'un isocyanate.